# EUROPÄISCHE PATENTANMELDUNG

(11) **EP 3 023 131 A1**
(43) Veröffentlichungstag der Anmeldung: **25.05.2016**
(21) Anmeldenummer: 14193677.3
(22) Anmeldetag: 18.11.2014
(51) Int. Cl.: B01D 15/00, C07C 7/12, C10G 25/00

(54) **Zweistufige Feinentschwefelung von Olefingemischen**

(71) Anmelder: Evonik Degussa GmbH, 45128 Essen (DE)
(72) Erfinder: Peitz, Stephan, 45739 Oer-Erkenschwick (DE); Geilen, Frank, 45721 Haltern am See (DE); Stochnol, Guido, 45721 Haltern am See (DE)

(57) **Zusammenfassung**

Bei der Reinigung von Kohlenwasserstoffgemischen wird ein verunreinigtes Kohlenwasserstoffgemisch enthaltend Olefine mit drei bis acht Kohlenstoffatomen in der Flüssigphase durch Kontakt mit einem ersten festen Sorptionsmittel zumindest teilweise von Verunreinigungen befreit. Der Erfindung lag die Aufgabe zu Grunde, ein Verfahren anzugeben, bei welchem im Gemisch vorhandene Schwefelverbindungen nahezu vollständig entfernt werden, ohne dabei wieder neue Schwefelverbindungen zu bilden. Nach der Reinigung soll ein Schwefelgehalt von unter 1 ppm gewährleistet werden, sodass nachfolgende katalytische Prozesse nicht vergiftet werden. Zudem soll im Gemisch enthaltendes 1-Buten nicht verloren gehen, denn das Verfahren soll sich dazu eignen, auch solche Kohlenwasserstoffgemische zu reinigen, die einen sehr geringen Anteil an wertvollem 1-Buten haben. Gelöst wird diese Aufgabe durch eine zweistufige Reinigung mit zwei unterschiedlichen Sorptionsmitteln, wobei es sich bei dem ersten Sorptionsmittel um ein CuO/ZnO/Al2O3-System handelt und bei dem zweiten Sorptionsmittel um ein Ni/ZnO/SiO2-System.

## Beschreibung

Die Erfindung betrifft ein Verfahren zur Reinigung von Kohlenwasserstoffgemischen, bei welchem ein verunreinigtes Kohlenwasserstoffgemisch enthaltend Olefine mit drei bis acht Kohlenstoffatomen durch Kontakt mit einem ersten festen Sorptionsmittel zumindest teilweise von Verunreinigungen befreit wird, wobei sich das Kohlenwasserstoffgemisch während des Kontakts mit dem ersten festen Sorptionsmittel ausschließlich im flüssigen Zustand befindet.

Kohlenwasserstoffe sind Verbindungen, die ausschließlich aus Kohlenstoff und Wasserstoff bestehen. Die Nomenklatur der Kohlenwasserstoffe basiert auf der Anzahl der pro Molekül des Kohlenwasserstoffes enthaltenden Kohlenstoffatome. In Kurzschreibweise wird gern das Präfix Cₙ verwendet, wobei n für die besagte Anzahl steht.

C₄-Kohlenwasserstoffe sind folglich Verbindungen, die ausschließlich aus Kohlenstoff und Wasserstoff bestehen, wobei die Anzahl der Kohlenstoffatome je Molekül vier beträgt. Wichtige Vertreter der C₄-Kohlenwasserstoffe sind die Alkene und Alkane mit vier Kohlenstoffatomen.

Alkene (synonym: Olefine) sind Kohlenwasserstoffe, die eine C=C Doppelbindung im Molekül aufweisen. Alkane (Paraffine) sind dagegen Kohlenwasserstoffe, die nur Einfachbindungen aufweisen. Sie werden deswegen auch als gesättigt bezeichnet. Alkane und Alkene mit derselben Anzahl von Kohlenwasserstoffen kommen in Rohstoffgemischen meist gemeinsam vor. Da Alkene aufgrund ihrer ungesättigten Doppelbindung reaktiver sind, eignen sie sich als Ausgangsstoff für chemische Reaktionen. Die weitaus reaktionsärmeren Alkane lassen sich in der Regel nur als Brennstoff einsetzen. Aufgrund der höheren Reaktivität sind Olefine wertvoller als Paraffine. Je größer der Anteil an Alkenen in einem Rohstoffgemisch gegenüber den Alkanen ist, desto teurer ist der Rohstoff.

Gemische aus C₄-Kohlenwasserstoffen sind Rohstoffe der Petro-Folgechemie. Sie stammen z.B. aus Streamcrackern (so genanntes "Crack C4"), aus katalytischen Crackern (so genanntes "FCC C4" (FCC: "fluid catalytic cracking") oder "DCC C4" (DCC "deep catalytic Cracking), aus Pyrolysen ("Pyrolyse C4"), aus MTO- bzw. MTP-Prozessen (MTO: "methanol to olefins", MTP: methanol to propylene) oder Dehydrierungen von Isobutan und n-Butan. Am verbreitesten sind C₄-Kohlenwasserstoffe aus Streamcrackern (Crack C4) und aus katalytischen Crackern (FCC C4). Es werden auch Gemische von C₄-Gemischen unterschiedlicher Herkunft gehandelt, so genannter "C₄-Schnitt". Zum Zwecke der Verwertung der einzelnen Komponenten sind die C₄-Gemische möglichst sortenrein in ihre Bestandteile zu zerlegen.

Die Aufarbeitung von C₄-Strömen aus Steamcrackern oder katalytischen Crackern wird prinzipiell beschrieben in K.-D. Wiese, F. Nierlich, DGMK-Tagungsbericht 2004-3, ISBN 3-936418-23-3. Eine ausführliche Gesamtprozessbeschreibung findet sich in DE102008007081A1.

Die für diese Erfindung relevanten Aspekte der C₄-Aufarbeitung werden im Folgenden kurz umrissen.

Technische C₄- Kohlenwasserstoffgemische aus den oben beschriebenen Quellen enthalten üblicherweise neben gesättigten und einfach ungesättigten Verbindungen auch mehrfach ungesättigte Verbindungen. Bevor einzelne Verbindungen aus diesen Gemischen isoliert werden können, ist es häufig notwendig, andere Verbindungen möglichst vollständig zu entfernen. Dies kann durch physikalische Methoden, wie z. B. Destillation, Extraktivdestillation oder Extraktion, aber auch durch eine selektive chemische Umsetzung der zu entfernenden Komponenten erfolgen. Besonderes Augenmerk muss dabei auf der möglichst vollständigen Entfernung von den im C₄-Kohlenwasserstoffgemisch enthaltenden Verunreinigungen, wie Sauerstoff-, Stickstoff- und Schwefelhaltigen Komponenten liegen, da diese als Katalysatorgifte negative Auswirkungen auf die einzelnen Prozessschritte haben können. Während diese Verunreinigungen typischerweise in Crack C4 nur in Spuren vorhanden sind, können diese z.B. in FCC C4-Strömen auch in höheren Konzentrationen vorhanden sein.

C₄-Kohlenwasserstoffgemische aus Steamcrackern oder Fluidized Catalytic Crackern weisen typischerweise die in Tabelle 0 aufgeführten Hauptkomponenten auf (Verunreinigungen nicht dargestellt).

**Tabelle 0: Typische Zusammensetzungen von Crack C4 und FCC C4**

| **Komponente** | **Crack C4** | **FCC C4** |
|---|---|---|
| | [Gew.-%] | [Gew.-%] |
| Isobutan | 1 - 3 | 15 - 45 |
| n-Butan | 6 - 11 | 5 - 15 |
| 1-Buten | 14 - 20 | 5 - 20 |
| 2-Butene | 4 - 8 | 20 - 35 |
| Isobuten | 20 - 28 | 10 - 20 |
| 1,3-Butadien | 40 - 45 | kleiner 1 |

Die Zusammensetzung der Rohstoffe kann je nach Herkunft des Materials stark schwanken. Zu den aufgeführten C₄-Komponenten gesellen sich noch Kohlenwasserstoffe mit weniger oder mehr Kohlenstoffatomen, sowie Verunreinigungen wie Mercaptane, Sulfide, Disulfide, Stickstoff- und sauerstoffhaltige Verbindungen in geringen Mengen.

Die Aufarbeitung von FCC C4 kann in einer Variante so erfolgen, dass zunächst die Konzentration des Isobutans mittels eines destillativen Schrittes in einer Destillation auf einen Wert von kleiner 5 Gew.-% gesenkt wird. Gleichzeitig werden die im Gemisch vorhandenen Leichtsieder (zum Beispiel C₃-Kohlenwasserstoffe, leichte Sauerstoff, Stickstoff- und Schwefel enthaltende Verbindungen) entfernt bzw. minimiert. Im darauf folgenden Schritt werden in einer Kolonne alle Hochsieder (zum Beispiel C₅-Kohlenwasserstoffe, schwere Sauerstoff-, Stickstoff- und Schwefel enthaltende Verbindungen) über den Sumpf entfernt. Im nächsten Schritt wird Isobuten entfernt, z. B. indem es mit Methanol zu Methyl-tert.-butylether (MTBE) umgesetzt und dieser durch Destillation entfernt wird. Soll reines Isobuten gewonnen werden, kann der Methyl-tert.-butylether anschließend wieder zu Isobuten und Methanol gespalten werden.

Zur weiteren Aufarbeitung des C₄-Gemisches müssen die noch verbliebenen mehrfach ungesättigten Verbindungen mit Hilfe eines Selektivhydrierprozesses zu den entsprechenden einfach ungesättigten und gesättigten Verbindungen umgesetzt werden. Jetzt können 1-Buten und verbliebenes Isobutan in ausreichender Reinheit destillativ abgetrennt und die verbleibenden 2-Butene und das n-Butan weiter aufgearbeitet werden. Häufig werden die 2-Butene durch Oligomerisierung, genauer gesagt, durch Dimerisierung zu Oktenen umgesetzt. Dabei wird aus zwei Molekülen mit je vier Kohlenstoffatomen ein Molekül mit acht Kohlenstoffatome aufgebaut. Die Oktene können anschließend mittels Hydroformylierung zu PVC-Weichmacheralkoholen umgesetzt werden. Die nach Abreaktion der Olefine verbleibenden gesättigten C4-Kohlenwasserstoffe können insbesondere als Treibmittel für Aerosole verwendet werden.

Unter einer Oligomerisierung ist ein Prozess zu verstehen, bei dem aus Olefinen wie insbesondere aus Propen und Butenen, höhere Alkene mit 6-20 Kohlenstoffatomen aufgebaut werden. Industriell angewendet wird beispielsweise der Nickel-katalysierte OCTOL® - Prozess, welcher in Hydrocarbon Process., Int. Ed. (1986) 65 (2. Sect.1), Seiten 31 bis 33 sowie in DE3914817, EP1029839 und DE102004018753 näher beschrieben ist.

Die für die einzelnen Verfahrensschritte verwendeten Einsatzströme haben durch vorangehende Prozesse, in denen immer wieder Verunreinigungen entfernt wurden, in der Regel bereits einen hohen Grad an Reinheit erreicht. Verbliebene Verunreinigungen können jedoch die Katalysatoren reversibel oder auch irreversibel desaktivieren. Diese Desaktivierung soll aus wirtschaftlichen Gründen natürlich auf ein Minimum reduziert werden. Daher sollten so viele Katalysatorgifte wie möglich durch weitere Reinigungsstufen vom Katalysator ferngehalten werden.

Die verschiedenen in den technischen C₄-Gemischen vorhandenen Katalysatorgifte wirken in unterschiedlicher Weise vergiftend. So werden die sauren Katalysatorsysteme oder Systemkomponenten wie Cokatalysatoren fast ausschließlich durch Komponenten vergiftet, die selbst basisch sind oder zumindest durch Folgereaktionen Basen freisetzen. Ein besonders typisches Beispiel für solche Stoffe ist Acetonitril, das als sehr schwache Base vergleichsweise schwer durch Sorptionsprozesse abgetrennt werden kann. Es vergiftet jedoch reversibel starke Lewis-Säuren. In Anwesenheit von Spuren von Wasser hydrolysiert es dort über Acetamid zur starken Base Ammoniak, das dann auch Brönsted-Säuren durch Bildung von Ammoniumionen irreversibel desaktiviert. Ein partielles Katalysatorgift stellt im Übrigen immer auch Wasser selbst dar, dessen Wirkung jedoch in der Regel reversibel ist, sofern es nicht durch weitere Reaktionen zur Bildung stärkerer Katalysatorgifte beiträgt. Für die Nickel-katalysierte Oligomerisierung von Butenen am OCTOL®-Katalysator führt bereits ein Wassergehalt von ca. 5 ppm zu messbarer Desaktivierung. Das Wasser wird jedoch von vielen Systeme an Olefine addiert und die gebildeten Alkohole werden durch die üblichen Katalysatorsysteme über eine Transferhydrierung unter Hydrierung von anderen ungesättigten Komponenten so lange oxidiert, bis das thermodynamische Gleichgewicht erreicht ist.

Auch die Metallkomplexkatalysatoren sind gegen basische Substanzen empfindlich. Die Vergiftungswirkung erfolgt dabei primär zumeist über die Desaktivierung des sauren Cokatalysators.

Die Metallkomponente der Katalysatoren wird hingegen besonders stark durch Komponenten wie Schwefel in Form bestimmter Verbindungen angegriffen, der unter bestimmten Umständen das Metallhydrid oder den Metallkomplex durch Bildung schwerlöslicher Sulfide irreversibel zerstört. Da die Metalle in der Regel in sehr niedrigen Oxidationsstufen vorliegen, sind besonders wirkungsvoll Schwefelverbindungen, die die Metalle zu einer relativ hohen Oxidationsstufe zu oxidieren vermögen, wie zum Beispiel Di- und Polysulfide. Unterschiedliche Schwefelverbindungen vermögen also primär durchaus unterschiedlich zu wirken. Während zum Beispiel Disulfide extrem gut abreagieren zu Thioethern und Schwefel, der dann die Metallhydride unter Bildung von Sulfiden oxidiert, wirken Thioether selbst primär zunächst wohl nur als Lewis-Base. Durch weitere, in der Regel im Detail gar nicht bekannte Prozesse und Reaktionen mit weiteren Spurenkomponenten des Systems führen auch sie aber letztlich auch - wenn auch wesentlich langsamer - zur Bildung von Metallsulfiden.

Nach dem vorangehend gesagten ist also für einen wirtschaftlich erfolgreichen Betrieb einer Anlage zur Zerlegung von Kohlenwasserstoffgemischen in seine Wertbestandteile mit Hilfe katalytischer Reaktionseinheiten die Aufgabe gestellt, die eingesetzten Katalysatoren möglichst effektiv vor Katalysatorgiften und insbesondere vor Schwefelverbindungen zu schützen. Dies trifft umso stärker zu, je mehr Edukt der Katalysator spezifisch umsetzen soll, in besonderem Maße also auf heterogene Katalysatoren wie den des OCTOL®-Prozesses.

In der betrieblichen Praxis entfernen alkalische Wäschen schwefelhaltige Giftstoffe aus Propen-und Butenströmen. Dabei reagieren Schwefelwasserstoff und Mercaptane besonders gut. In der Regel werden die alkalischen Waschlösungen durch Oxidation mit Luft regeneriert.

Ein solches Waschverfahren wird für den industriellen Einsatz von der Fa. UOP LLC unter dem Namen MEROX® angeboten. (G. A. Dziabis, "UOP MEROX PROCESS" in Robert Meyers, Handbook of Petroleum Refining Processes, 3rd Edition, 2004 McGraw-Hill).

Beim MEROX®-Verfahren werden die Mercaptane in der wässrigen Waschlösung zu Di- und Polysulfiden oxidiert, die als ölige Phase abgetrennt werden. Ein kleiner Teil dieser Di- und Polysulfide verbleibt jedoch in der wässrigen Alkalilauge gelöst oder suspendiert, und es gelingt auch durch eine Wäsche dieser wässrigen Phase mit einem Waschöl oder ähnlichem oft nicht, diesen Rest vor der Rückführung in die Wäsche quantitativ zu entfernen, so dass zwar die Mercaptane weitgehend entfernt, auf der anderen Seite aber kleine Mengen an Di- und Polysulfiden wieder in den Strom eingetragen werden. Diese stellen, wie eben erwähnt, Schwefelkomponenten dar, die die für die Reaktion wesentlichen Metallhydride in schwerlösliche Metallsulfide umwandeln und den Katalysator dadurch irreversibel desaktivieren. Typischerweise enthalten zum Beispiel die Ströme an FCC C4 etwa 100 bis 200 ppm Schwefel. Nach der MEROX®-Wäsche ist dieser Gehalt dann üblicherweise bis auf einen Wert von unter 10 ppm reduziert, wobei die Schwefelverbindungen dann überwiegend aus den genannten Di- und Polysulfiden, aber auch aus höheren Mercaptanen bestehen.

In der Praxis kann man einen Teil der Gifte auch durch geschickte Anordnung von Trennoperationen, wie zum Beispiel Destillationen, in Fraktionen lenken, in denen sie nicht mehr mit empfindlichen Katalysatoren in Berührung kommen. Häufig ist dies aber nicht in dem Umfange möglich, der in Hinblick auf die Reinheit der Ströme wünschenswert erscheint, so dass vor den Katalysatorbetten Sorptionsmittel vorgeschaltet werden müssen, um die erforderliche Reinheit zu gewährleisten.

Sorptionsmittel sind feste Stoffe, die in der Lage sind, einen anderen Stoff, das so genannte Sorbat, an sich zu binden, sofern sie mit dem Sorbat in Kontakt kommen. Die Bindung erfolgt an der Oberfläche des Sorptionsmittels durch physikalische und/oder chemische Effekte. Insoweit unterscheidet man physikalische und chemische Adsorption. Da die Wirkweise eines Sorptionsmittels nicht immer zweifelsfrei feststeht, wird hier wirkungsneutral von einem Sorptionsmittel gesprochen.

Aus technischer Sicht sind Sorptionsmittel allgemein zu unterscheiden in solche, die regenerierbar sind und in solche, die die Katalysatorgifte irreversibel umwandeln bzw. chemisch binden.

Als regenerierbare Sorptionsmittel kommen häufig Molekularsiebe und Zeolithe zum Einsatz. Die regenerierbaren Sorptionsmittel binden Verschmutzungen mit nur moderater Festigkeit. Im Zuge der Regenerierung des Sorptionsmittels werden Bedingungen wie zum Beispiel höheren Temperaturen und niedrigere Drücke eingestellt, unter denen das Sorptionsmittel das Sorbat wieder frei lässt. Diese Eigenschaft führt zu einer relativ geringen Kapazität bis zum Durchbruch. Zusätzlich entstehen oft hohe Betriebskosten durch Freistellen und Spülen des Sorptionsmittels sowie durch die Bereitstellung und Entsorgung der Regeneriergase oder auch der Flüssigkeitsströme.

Irreversible Sorptionsmittel werden dagegen nicht regeneriert sondern nach ihrem Durchbruch entsorgt. Sie müssen daher preiswert verfügbar und entsorgbar sein. Da irreversiblen Sorptionsmittel das Adsorbat chemisch binden, ist ihre Durchlässigkeit gegenüber den zu adsorbierenden Stoffen geringer als bei regenerierbaren Sorptionsmitteln. Irreversible Sorptionsmittel erzielen deswegen bessere Reinheitsgrade als regenerierbare Sorptionsmittel.

EP 0 064 464 A1 beschreibt Kontaktmassen, welche insbesondere zur Entschwefelung von Kohlenwasserstoffchargen verwendbar sind. Die Kontaktmassen enthalten Kupferoxid und basieren auf einem Träger aus Tonerde oder Zeolith von Typ X oder Y. Bedenklich ist der zwingend erforderliche Gehalt an Cadmiumoxid, da Cadmium als kanzerogen eingestuft ist. Kanzerogene Stoffe lassen sich nur aufwendig handhaben und entsorgen, sodass insbesondere der irreversible Einsatz derartiger Kontaktmassen unwirtschaftlich ist.

EP 0 354 316 B1 beschreibt die cadmiumfreie Feinentschwefelung von flüssigen C₄-Kohlenwasserstoffgemischen an Kupfer, Silber und Zink enthaltenden Zeolithen. Der bevorzugte Temperaturbereich liegt zwischen 50 und 130°C, der bevorzugte Druck bei 1 bis 50 bar. Die Raum/Zeit-Belastung wird mit 1 bis 40 h⁻¹ angegeben. Wenngleich das hier beschriebene Sorptionsmittel kein bedenkliches Cadmium enthält, ist dieses Material aufgrund seines hohen Silbergehalts von mindestens 2 Gew.-% ebenso unwirtschaftlich.

Besonders anfällig gegen Katalysatorgifte sind Nickel-haltige Oligomerisierungskatalysatoren. Kohlenwasserstoffgemische mit zwei bis vier Kohlenstoffatomen dienen oft als Substrat für Oligomerisierungen wie dem OCTOL®-Prozess. Um Katalysatorgifte effektiv zu entfernen hat sich bewährt, solche Ströme vor dem Eintritt in die Oligomerisierung über ein Molekularsieb zu leiten. So beschreibt etwa EP0395857B1 ein Verfahren, bei welchem eine Entschwefelung von Raffineriepropen vor dessen Oligomerisierung an einem mit Kupfer ausgetauschten X-Zeolithen bei einer Temperatur von 120°C, einem Druck von 50 bar abs. und einer Raum/Zeit-Belastung von 0.75 h⁻¹ erfolgt. Bei diesen Bedingungen ist Propen überkritisch.

Da diese einfachen Molekularsiebe leicht verfügbar und gesundheitlich unbedenklich sind, stellen sie in der industriellen Praxis heute das Sorptionsmittel der Wahl zur Feinentschwefelung von C₃-bis C₈ - Kohlenwasserstoffgemischen dar. Da die Molekularsiebe die Verunreinigungen nur physikalisch binden, lassen sich derartige Sorptionsmittel regenerieren. Allerdings ist ihr Sorptionsvermögen jedoch im Vergleich zu chemisch wirkenden Sorptionsmitteln geringer, sodass mit der Feinentschwefelung an Zeolithen nur mäßige Reinheiten zu erzielen sind.

Aus der WO2014/009159A1 ist die Verwendung von pyrophorem Nickel zur Schwefeladsorption vor Oligomerisierungsanlagen bekannt. In einem Versuch wird dasselbe Sorptionsmittel in zwei in Reihe geschaltete Reinigungsbetten angeordnet und erfolgreich zur Entfernung von Dimethyldisulfid aus einem C₄-Olefingemisch genutzt. Allerdings zeigen neuere Untersuchungen mit verbesserter Analysetechnik, dass dieses Sorptionsmittel lediglich 75 % des eingetragenen Schwefels entfernt, da 25 % der Schwefelfracht den Adsorber in Form von Sulfiden wieder verlässt. Die Sorption ist somit nicht sehr effizient. Prinzip bedingte Nachteile dieses Materials sind außerdem seine pyrophoren Eigenschaften, die seine Handhabung erschweren sowie das Erfordernis, dieses Sorptionsmittel mit Wasserstoff zu aktivieren.

In der zum Anmeldezeitpunkt noch unveröffentlichten deutschen Patentanmeldung 102013225724.4 ist die Reinigung flüssiger Olefingemische mit Hilfe eines Kupfer/Zink/Aluminiumkatalysators beschrieben, wie er normalerweise in der Methanolsynthese eingesetzt wird. Versuche belegen, dass dieses Material alle üblicherweise in C₄-Olefingemischen vorkommenden Schwefelverbindungen (insbesondere Mercaptane) nahezu vollständig bindet. Gegenüber dem pyrophoren Nickelmaterial hat es den Vorteil, dass es sich einfacher handhaben lässt und ohne eine Aktivierung einsetzbar ist. Ein Nachteil dieses Materials ist, dass es einen Teil des eingetragenen Schwefels in Form von Disulfiden (Dimethyldisulfid, Diethyldisulfid, Ethylmethyldisulfid u.ä.) wieder abgibt. Die Disulfidbildung findet im Adsorber selbst statt, das Material ist insoweit katalytisch aktiv. Möglicherweise werden auf der CuO-Oberfläche zwei Thiolat-Einheiten oxidativ zu Disulfiden verknüpft. Dies geschieht allerdings nur in geringem Maße, wie aus der DE102013225724.4 ersichtlich. Ein weiterer Nachteil der CuO/ZnO/Al2O3-Systeme ist der im Vergleich zu den Ni-basierten Sorptionsmitteln die geringere Gesamtkapazität. Deswegen muss dieses Sorptionsmittel recht häufig ausgetauscht werden.

In Hinblick auf diesen Stand der Technik lag der Erfindung die Aufgabe zu Grunde, ein Verfahren zur Reinigung von flüssigen Olefingemischen anzugeben, bei welchem im Gemisch vorhandene Schwefelverbindungen nahezu vollständig entfernt werden, ohne dabei wieder in signifikantem Maße neue Schwefelverbindungen zu bilden. Nach der Reinigung soll ein Schwefelgehalt von unter 1 ppm gewährleistet werden, sodass nachfolgende katalytische Prozesse wie insbesondere Oligomerisierungen nicht vergiftet werden. Zudem soll mit der Reinigung möglichst kein Wertverlust einhergehen. Dieser wäre insbesondere dann zu befürchten, wenn im verunreinigten Gemisch enthaltendes, wertvolles 1-Buten zu weniger wertvollem 2-Buten isomerisiert werden würde.

Gelöst wird diese Aufgabe durch eine zweistufige Reinigung mit zwei unterschiedlichen Sorptionsmitteln, wobei es sich bei dem ersten Sorptionsmittel um ein CuO/ZnO/Al2O3-System handelt und bei dem zweiten Sorptionsmittel um ein Ni/ZnO/SiO2-System.

Bei der ersten Stufe handelt es sich um ein Kupferoxid/Zinnoxid -haltiges System auf Basis von Aluminiumoxid, wie es sich auch als Katalysator in der Methanolsynthese eignet. Als erstes festes Sorptionsmittel verwendet, werden hier die in technischen Olefingemischen gängigen Schwefelverbindungen wie insbesondere Mercaptane vollständig zurückgehalten. Die dabei unvermeidlich in niedriger Konzentration gebildeten Disulfide werden sodann mit dem zweiten Sorptionsmittel abgefangen. Bei dem zweiten Sorptionsmittel handelt es sich um ein Nickel/Zinnoxid-haltiges System auf Basis von Siliciumdioxid.

Dank der zweistufigen Reinigung kann hinter der zweiten Stufe ein Gehalt an schwefelhaltiger Substanzen von unter 1 ppm gewährleisten werden, was zu einer deutlich längeren Laufzeit von Oligomerisierungskatalysatoren führt.

Schließlich macht die Erfindung auch eine Selektivhydrierung von Butadien-haltigen Strömen überhaupt erst ermöglicht, ohne dabei 1-Buten zu verlieren: Es ist allgemein bekannt, dass geschwefelte Selektivhydrierkatalysatoren oder S-haltige Feeds dazu führen, dass parallel zur gewünschten Selektivhydrierung von Butadien zu Butenen auch 1-Buten hydroisomerisiert wird (vgl. EP1453775B1). Dies hat zur Folge, dass in den genannten Fällen kein 1-Butengehalt im Produktstrom erreicht werden kann, der signifikant oberhalb der thermodynamischen Gleichgewichtszusammensetzung der 1-und 2-Butene liegt.

Gegenstand der Erfindung ist mithin ein Verfahren zur Reinigung von Kohlenwasserstoffgemischen, bei welchem ein verunreinigtes Kohlenwasserstoffgemisch enthaltend Olefine mit drei bis acht Kohlenstoffatomen durch Kontakt mit einem ersten festen Sorptionsmittel und mit einem zweiten festen Sorptionsmittel zumindest teilweise von Verunreinigungen befreit wird, bei welchem sich das Kohlenwasserstoffgemisch während des Kontakts mit beiden Sorptionsmitteln ausschließlich im flüssigen Zustand befindet,
wobei das erste feste Sorptionsmittel die folgende, sich zu 100 Gew.-% ergänzende Zusammensetzung aufweist:
- Kupferoxid: 10 bis 60 Gew.-%;
- Zinkoxid: 10 bis 60 Gew.-%;
- Aluminiumoxid: 10 bis 30 Gew.-%;
- sonstige Stoffe: 0 bis 5 Gew.-%;
und wobei das zweite feste Sorptionsmittel die folgende, sich zu 100 Gew.-% ergänzende Zusammensetzung aufweist:
- Nickel: 15 bis 65 Gew.-%;
- Zinkoxid: 0 bis 40 Gew.-%;
- Siliciumdioxid: 5 bis 75 Gew.-%;
- Graphit: 0 bis 5 Gew.-%;
- sonstige Komponenten 0 bis 40 Gew.-%.

Die Grundidee der vorliegenden Erfindung besteht darin, die Reinigung in zwei Schritten vorzunehmen und dabei in dem ersten Schritt ein CuO/ZnO/Al₂O₃-Katalysator als Sorptionsmittel zu verwenden, wie er normalerweise in der Methanolsynthese eingesetzt wird. Diese Idee ist bereits in der der zum Anmeldezeitpunkt noch unveröffentlichten deutschen Patentanmeldung 102013225724.4 niedergelegt. Allerdings wurde dabei noch nicht erkannt, dass derartige Sorptionmittel Disulfide bilden. Diese lassen sich allerdings hervorragend mit einem zweiten Sorptionsmittel auf Ni/ZnO/SiO₂-Basis entfernen. Dieses aus der WO2014/009159A1 bekannte Material muss zwar mit Wasserstoff aktiviert werden, was aber in seiner Position als zweite Reinigungsinstanz nicht so sehr nachteilig ist, da es kaum beladen und deswegen nur selten aktiviert werden braucht. Die Vorteile beider Systeme werden mithin synergetisch genutzt.

Die hier genutzte Verwendbarkeit von Methanol-Katalysatoren auf CuO/ZnO/Al₂O₃-Basis zur Entgiftung von Kohlenwasserstoffgemischen ist deshalb überraschend, weil die Methanol-Synthese stets in Anwesenheit von Wasserstoff erfolgt, währenddessen Wasserstoff in den zu entgiftenden Stoffströmen in der Regel nicht nennenswert enthalten ist. So sind marktübliche Crack C4 - und FCC C4-Ströme frei von Wasserstoff (< 1 Gew.-ppm). Die Entgiftung solcher Ströme erfolgt quasi in Abwesenheit von Wasserstoff.

Des Weiteren erfolgt die Aufarbeitung von C₃ - bis C₈ -Kohlenwasserstoffgemischen in der Regel in der Flüssigphase, da sich die Kohlenwasserstoffe mit mehr als zwei Kohlenstoffatomen mit geringem Aufwand verflüssigen und dann mit einer hohen Prozessintensität verarbeiten lassen. Die Methanol-Synthese indes erfolgt ausschließlich in der Gasphase. Es war nicht zu erwarten, dass für die Gasphasenkatalyse bestimmten Materialen sich auch zur Flüssigphasensorption eignen würden.

Grundsätzlich eignet sich jeder kommerziell verfügbare CuO/ZnO/Al2O3-Katalysator als erstes festes Sorptionsmittel zur Reinigung der C₃- bis C₈-Kohlenwasserstoffgemische. Bevorzugt werden jedoch solche Katalysatoren als erstes festes Sorptionsmittel eingesetzt, welche die folgende Zusammensetzung aufweisen:
- Kupferoxid: 30 bis 45 Gew.-%;
- Zinkoxid: 30 bis 50 Gew.-%;
- Aluminiumoxid: 10 bis 15 Gew.-%;
- weitere Metalloxide: 0 bis 2 Gew.-%;
- Graphit: 0 bis 3 Gew.-%;
- andere Stoffe: 0 bis 1 Gew.-%.

Wie aus den Gewichtskonzentrationen ersichtlich, sind Kupfer-, Zink- und Aluminiumoxid die maßgeblichen Bestandteile des ersten festen Sorptionsmittels. Das Al₂O₃ dient als Trägermaterial, welches an seiner Oberfläche mit CuO und ZnO belegt ist. Als weitere enthaltene Metalloxide kommen beispielsweise Eisenoxide oder Magnesiumoxide in Betracht. Schwermetalloxide, die bekanntermaßen gesundheitsgefährdend sind, wie beispielsweise Cadmium oder Blei oder Chrom, sollte nach Möglichkeit nicht enthalten sein. Geringere Mengen an Graphit oder Magnesiumstearat dienen als Bindemittel und Verarbeitungshilfsmittel bei der Formgebung des Sorptionsmittels. Unter "anderen Stoffen" sind produktionsbedingte Verunreinigungen des Sorptionsmittels zu verstehen.

Hinsichtlich der Formgebung kann das erste feste Sorptionsmittel in Pulverform oder als Granulat vorliegen. Darüber hinaus kann das Sorptionsmittel in eine makroskopische Form gepresst sein, wie beispielsweise in Kugeln, oder in Pellets oder in Ringe.

Die Verwendung von Material mit einer großen Kupferoxidoberfläche ist vorteilhaft, weil die Reaktionsgeschwindigkeit der Adsorption und der Umwandlung mit ihr korreliert und diese Materialien auch eine höhere Sorptionskapazität aufweisen. Bevorzugt weist das erste Sorptionsmittel eine Kupferoxidoberfläche von mindestens 50 m²/g, bevorzugt 100 m²/g, bezogen auf seinen Kupferoxidgehalt auf. Dies begünstigt die Sorptionswirkung. Die Oberfläche wird per Stickstoff-Sorption bestimmt.

Die Herstellung des ersten Sorptionsmittels geeignet sind grundsätzlich alle technischen Methoden, die zu einem Festkörper führen, welcher eine ausreichende Festigkeit zur Handhabung besitzt. Sie umfasst im Wesentlichen die beiden Arbeitsschritte:
i) Bereitstellen eines porösen Gerüstmaterials aus Aluminiumoxid und/oder Graphit;
ii) Vermengen des Gerüstmaterials mit Kupferoxid und Zinkoxid.

Eingesetzt werden können Kupferoxidpulver, Kupfercarbonatpulver oder hydroxidhaltige Kupferverbindungen sowie Gemische daraus. Im Falle des Kupfers kann auch eine kupfercarbonathaltige Verbindung mit Hilfe einer ammoniakalischen Lösung ganz oder teilweise in eine Kupfertetramincarbonatlösung überführt werden, die als Einsatzmaterial dient. Diese Substanzen werden entsprechend den erfindungsgemäßen Mischungsverhältnissen zusammen mit Zinkoxid, Zinkcarbonat oder Zinkhydroxid sowie einem Al₂O₃-haltigem Pulver vermischt. Anstelle von Al₂O₃ kann auch teilweise SiO₂ eingesetzt werden. Als Al₂O₃-haltiges Pulver können alle Modifikationen von Al₂O₃ eingesetzt werden sowie auch Aluminiumoxidhydrat oder Aluminiumhydroxyoxide sowie Aluminiumhydroxid. Die einzelnen Feststoffkomponenten können in geeigneten Mischern, Intensivmischern oder Knetern vermengt und homogenisiert werden. Dabei ist es üblich, eine Befeuchtung mit entmineralisiertem Wasser vorzunehmen. Nach ausreichender Mischung kann eine beliebig geeignete Formgebung erfolgen. Unter Umständen ist eine vorhergehende ganz oder teilweise Trockung und/oder Mahlung der Mischung nötig. Für die Formgebung sind beispielsweise Extruder oder Tablettenpressen geeignet. Auch Pelletierteller können für diese Zwecke dienlich sein. Im Falle einer Tablettierung wird dem Gemisch oftmals ein Gleithilfsmittel wie Graphit zugeführt. Im Falle einer Extrusion werden oft andere organische Zusätze gewählt, die geeignet sind die notwendige Plastifizierbarkeit des Gemisches einzustellen. Dazu zählen beispielsweise zelluloseartige Substanzen, Polyether, Polyethylenglycol und andere, die unter Umständen auch als Porenbildner dienen können, wenn die Substanzen durch eine thermische Behandlung, die sich der Formgebung in der Regel anschließt, ganz oder teilweise entfernt werden. Im Falle einer Pelletierung auf einem entsprechenden Pelletierteller wird die Aufbauagglomeration durch die langsame Zugabe einer geeigneten Menge an Wasser erreicht.

Die thermische Behandlung wird in einem Schritt oder in sequentiellen Schritten durchgeführt. Hierbei werden Wasseranteile oder aber organische Anteile entfernt und die mechanische Festigkeit des Formkörpers wird in der Regel dabei erhöht. Außerdem werden die notwendigen Oxidphasen ausgebildet, wenn die Vorstufenmaterialien noch nicht in dieser Form vorlagen.

In einer anderen Art der Herstellung werden Nitratsalze in wässriger Lösung eingesetzt oder die oxydischen Verbindungen werden mit Salpetersäure ganz oder teilweise gelöst. Insbesondere Im Fall der aluminiumoxidischen Verbindungen löst man oftmals nicht vollständig auf sondern modifiziert das Material mit Hilfe der Säure, dieser Vorgang wird als Peptisierung bezeichnet. Das Peptid wird dann mit den anderen gelösten Komponenten wie oben beschreiben vermischt und zu einem Formkörper verarbeitet. Die Temperung führt dann dazu, dass sich aus den Nitraten die jeweiligen Oxide ausbilden können, wenn die Temperatur geeignet gewählt wurde.

Die Verwendung von nitrathaltigen Salzlösungen kann auch dazu führen, dass eine Fällungsreaktion durchgeführt werden muss, um zu einem Feststoffgemisch zu kommen. Die pH-Einstellung erfolgt mit Natronlauge- oder Sodalösungen. Beispiele hierzu finden sich in US4535071.

Weiterhin ist es möglich, Nitratsalzlösungen mittels Sprühtrocknung in ein oxydisches Produktgemisch als Feststoff zu überführen. In der Regel folgen dann eine Mahlung und eine wie zuvor beschriebene Formgebung. Eine abschließende Temperung, die aber auch direkt nach der Sprühtrockung oder der Mahlung der Bestandteile durchgeführt werden kann, führt die notwendige restliche Nitratzersetzung herbei und überführt die Komponenten hinzu den Oxiden und verfestigt den Formköper.

Die vorstehend beschriebene Eigenfertigung des Sorptionsmittels kann durch Verwendung eines kommerziell verfügbaren Methanol-Katalysators entfallen. Geeignet sind beispielsweise MegaMax® 700 und 800 von Clariant (vormals Süd-Chemie) und Haldor Topsoe's Mk-101 und Mk-121. Beschrieben sind diese Katalysatoren in Nitrogen + Syngas 290, November-December 2007, Seite 36.

In der Patentliteratur beschreiben DE2846614C3, DE1568864C3, EP0125689B2, DE10160486A1 und US4279781 Kupfer/Zink/Aluminiumkatalysatoren für die Herstellung von Methanol.

Das erfindungsgemäß eingesetzte zweite Sorptionsmittel umfasst ein poröses Trägermaterial (SiO₂), das auf seiner Oberfläche mit Nickel und Zinnoxid belegt ist. Graphit wird bei der Formgebung als Gleitmittel benötigt und ist daher ebenfalls enthalten. Weitere Komponenten können aus produktionstechnischen Gründen enthalten sein.

Als Trägermaterial wird vorzugsweise Fällungskieselsäure verwendet. Da diese sehr fein ist und eine große spezifische Oberfläche aufweist, führt die große Oberfläche an elementarem Nickel zu pyrophoren Eigenschaften, das heißt, dass sich das zweite feste Sorptionsmittel bei 20°C und Luftatmosphäre selbst entzündet. Dies erschwert zwar die Handhabung des zweiten festen Sorptionsmittels, steigert aber seine Effektivität. Die Verwendung von pyrophorem Material ist deswegen eine Frage des Verunreinigungsgrades und der geforderten Reinheit. Pyrophores Material sollte nur dann verwendet werden, wenn die Reinigungsaufgabe dies erfordert, was aber oft der Fall sein wird. Deswegen ist die Verwendung von einem auf Ni/ZnO/SiO2-System mit pyrophoren Eigenschaften als zweites festes Sorptionsmittel bevorzugt.

Pyrophores Material erhält man dadurch, dass man eine metallische Nickeloberfläche von mindestens 3 m²/g bezogen auf den Nickelgehalt des zweiten festen Sorptionsmittels vorsieht. Dies begünstigt die Adsorptionswirkung und bedingt zugleich die pyrophoren Eigenschaften. Die Oberfläche wird mittels Wasserstoff-Chemiesorption gemessen. Optimal liegt besagte Nickeloberfläche bei etwa 9 m²/g.

Bevorzugt wird also ein zweites Sorptionsmittel eingesetzt, das eine metallische Nickeloberfläche von größer als 3 m²/g, bevorzugt etwa 9 m²/g aufweist, jeweils bezogen auf das Gesamtgewicht an Nickel in dem zweiten Sorptionsmittel.

Um das Ni/ZnO/SiO2-System zu aktivieren, ist es vor Gebrauch mit einem Wasserstoffstrom bei einer Temperatur von 150°C bis 400°C, bevorzugt 180°C bis 280°C, insbesondere 200°C bis 240°C zu spülen. Im Betrieb ist kein weiterer Energieeintrag erforderlich. Die Aktivierung mit Wasserstoff kann in-situ oder ex-situ erfolgen, also am späteren Einsatzort oder davon entfernt, nach der Herstellung des zweiten festen Sorptionsmittels. Sofern das zweite Sorptionsmittel desaktiviert, kann es mit einer weiteren Wasserstoffspülung wieder reaktiviert werden.

Nach erfolgter Aktivierung wird der Wasserstoffstrom abgeschaltet. Im Gegensatz zur Methanol-Synthese wird das erfindungsgemäße Reinigungsverfahren in Abwesenheit von Wasserstoff durchgeführt. Eine 100%-ige Abwesenheit von Wasserstoff lässt sich in der industriellen Technik natürlich nicht gewährleisten. Unter der "Abwesenheit von Wasserstoff" soll deswegen ein Wasserstoffgehalt von unter 1 Gew.-ppm bezogen auf die Gesamtmasse des verunreinigten Kohlenwasserstoffgemisches verstanden werden. Die Maßeinheit ppm wird hier als 10⁻⁶ verstanden.

Als zweites festes Sorptionsmittel wird bevorzugt ein verfügbares Produkt eingesetzt. Überraschenderweise wurde gefunden, dass der von der Evonik Industries AG erhältliche Katalysator H 10126 rs ein hervorragendes zweites Sorptionsmittel für die Verwirklichung der vorliegenden Erfindung darstellt.

Ein besonderer Vorteil des Verfahrens liegt mithin darin, dass beide für die Reinigung erforderlichen Sorptionsmittel als Katalysatoren kommerziell verfügbar sind und deswegen nicht erst hergestellt werden müssen.

Sofern das zweite feste Sorptionsmittel nicht zugekauft werden soll, kann seine Herstellung grundsätzlich durch die folgenden Schritte geschehen:
w) Bereitstellen eines porösen Gerüstmaterials aus Siliciumdioxid;
x) Vermengen des Gerüstmaterials mit Nickelcarbonat und Zinkoxid;
y) Thermische Zersetzung des Nickelcarbonats zu NiO
z) Reduktion unter Wasserstoffzufuhr zu metallischem Nickel.

Schritte y) und z) können zur Vermeidung von Exothermiespitzen auch in einem Schritt erfolgen, da y) endotherm und z) stark exotherm sind.

In einer ersten Ausführungsform wird also in einem Schritt das eingesetzte Nickelcarbonat (NiCO₃) thermisch zersetzt und parallel entstehendes NiO mit Wasserstoff zu metallischem Nickel reduziert. In einer zweiten Ausführungsform können beide Schritte separat erfolgen. In beiden Fällen kann zur Aktivierung auch ein Gemisch aus Stickstoff und Wasserstoff verwendet werden, dessen Wasserstoffgehalt im Laufe der Aktivierung gesteigert wird.

Die Herstellung des Adsorptionsmittels entspricht im Wesentlichen der Herstellung von Katalysatoren mit ähnlicher Zusammensetzung.

Beide Sorptionsmittel werden vorzugsweise als Bett direkt vor dem zu schützenden Katalysator aufgeschüttet und von dem zu reinigenden Kohlenwasserstoffgemisch durchströmt. Um die richtige Reinigungsabfolge zu gewährleisten, wird das erste feste Sorptionsmittel in einem ersten Bett angeordnet ist und das zweite feste Sorptionsmittel in einem stromabwärts hinter dem ersten Bett gelegenen zweiten Bett. Auf diese Weise strömt das zu reinigende Kohlenwasserstoffgemisch nacheinander durch das erste und sodann durch das zweite Bett. Auf diese Weise ist sichergestellt, dass zuerst das CuO/ZnO/Al2O3-System durchlaufen wird und sodann das Ni/ZnO/SiO2-System.

Die beiden Betten sollten sich in einem anderen Gefäß befinden wie der zu schützende Katalysator. Grund dafür ist, dass das zweite Sorptionsmittel mit einem H₂-Strom reduziert werden muss. Die stromabwärts angeordneten Katalysatoren wie zum Beispiel ein Nickel-haltiger Oligomerisierungs-Katalysator dürfen hingegen nicht mit H₂ reduziert werden, weswegen separate Gefäße erforderlich sind.

Wichtig im Sinne der vorliegenden Erfindung ist, dass das Sorptionsmittel keine wesentliche katalytische Aktivität zur Hydrierung, Veretherung, Oligomerisierung oder weiteren Reaktionen von Olefinen aufweist. Die genannten Reaktionen der Kohlenwasserstoffe sollen ausschließlich an den dafür vorgesehenen Katalysatoren, nicht aber im Reinigungsbett erfolgen. Die zu schützenden Katalysatoren befinden sich deswegen vorzugsweise außerhalb des Reinigungsbettes, zumindest in einer anderen Schüttung oder in anderen Apparaten.

Je nach den Umständen werden üblicherweise Verweilzeiten zwischen 0.01 und 0.2 Stunden im Reinigungsbett vorgesehen, bei Bedarf aber auch darüber hinaus. Da der Betrieb bei erhöhter Temperatur die Abreaktion beschleunigt und die Schwefelkapazität erhöht, ist es vorteilhaft, sie nach den meist vorhandenen Vorwärmern anzuordnen. Die Einhaltung einer bestimmten Temperatur des Sorptionsmittels ist maßgeblich für sein Reinigungsvermögen. Versuche zeigen, dass der Kontakt deswegen bei Temperaturen zwischen 10°C und 150°C stattfinden soll, bevorzugt zwischen 20°C und 130°C und ganz besonders bevorzugt zwischen 30°C und 120°C. Die optimale Kontakttemperatur liegt zwischen 80°C und 100°C. Da kommerzielle Methanol-Katalysatoren bei deutlich höheren Temperaturen eingesetzt werden, ist Temperaturstabilität in diesen Bereichen gegeben. Falls der zu schützende Katalysator bei einer anderen Temperatur betrieben wird, sollte das Sorptionsmittel in einem separaten Gefäß, also außerhalb des Reaktors angeordnet werden.

Wichtig ist, dass sich das verunreinigte Kohlenwasserstoffgemisch während des Kontakts mit dem Sorptionsmittel ausschließlich im flüssigen Zustand befindet. Im angegebenen Temperaturbereich wird dies durch einen Druck zwischen 0.5 und 3.5 MPa (5 bis 35 bar) gewährleistet. Letztendlich kommt es auf den Druck jedoch nicht an, solange sich die Kohlenwasserstoffe im flüssigen Zustand befinden. Die Raum-/Zeit-Belastung (weight hour space velocity - WHSV) wird dann vorzugsweise zwischen 0.5 und 15 h⁻¹ gewählt. Dies bedeutet, dass pro Kilogramm Sorptionsmittel zwischen 0.5 und 15 Kilogramm pro Stunde verunreinigtes Kohlenwasserstoffgemisch über das Reinigungsbett gefahren wird. Die Betten bestehen aus einer Schüttung des jeweiligen Sorptionsmittels mit einer Schüttdichte im Bereich von 0.7 bis 1.5 kg/m³, bevorzugt etwa 1.15 kg/m³.

Die hier geschilderten Betriebsbedingungen sind für beide Sorptionsmittel vorteilhaft. Deshalb sollten beide Reinigungsschritte des erfindungsgemäßen Verfahrens bei diesen Parameterwerten durchgeführt werden. Es ist aber nicht zwingend erforderlich, dass die Betriebsbedingungen für beide Sorptionsmittel identisch sind. Vielmehr können erstes und zweites Sorptionsmittel auch bei unterschiedlichen Drücken, Temperaturen, Raum/Zeit-Belastungen und Schüttdichten durchströmt werden, solange die jeweiligen Werte in den bevorzugten Bereichen liegen. Sofern eine physikalische Mischung beider Sorptionsmittel als gemeinsames Reinigungsbett verwendet wird, sind die Betriebsbedingungen denknotwenig identisch.

Das erste Sorptionsmittel wird üblicherweise in einem oxidierten Zustand angeliefert, der eine Handhabung bei Raumtemperatur an der Luft zulässt. Nach dem Befüllen des Reaktors muss das erste Sorptionsmittel nicht mehr durch eine Nachreduktion aktiviert werden. Auch nach dem Einsatz muss das erste Sorptionsmittel nicht durch Oxidation mit Luft stabilisiert werden, so dass es in einfacher Weise aus dem Reaktor entnommen werden kann.

Das zweite Sorptionsmittel wird üblicherweise in oxidierter Form angeliefert, die eine Handhabung bei Raumtemperatur an der Luft zulässt. Nach dem Befüllen des Reaktors muss das zweite Sorptionsmittel durch eine Nachreduktion aktiviert werden. Nach dem Einsatz muss das zweite Sorptionsmittel durch Oxidation mit Luft stabilisiert werden, so dass es in einfacher Weise aus dem Reaktor entnommen werden kann.

Um eine besonders effektive Reinigung zu erreichen und Betriebsunterbrechungen durch Wechsel der Sorptionsmittel zu vermeiden, empfiehlt es sich, mehrere Behälter einzusetzen, die derart revolvierend in Reihe geschaltet werden können, dass am Eintritt immer der Behälter mit der höchsten und zu Austritt hin immer der mit der niedrigsten Beladung angeordnet ist. Mindestens ein Behälter kann dabei, ohne den zu reinigenden Strom zu unterbrechen, herausgenommen und das in ihm befindliche Material gespült und entnommen werden, danach erfolgt in analoger Weise die Neubefüllung.

Das erfindungsgemäße Verfahren eignet sich für die Aufreinigung aller

Kohlenwasserstoffgemische, die Olefine mit drei bis acht Kohlenstoffatomen aufweisen. Als technisch relevant sind davon zu erachten z.B. Propen, n-Butene, n-Pentene, Hexene, Neohexen etc sowie deren gesättigte Analoga. Von ihnen nehmen Propan/Propen und die Butane/Butene die absolut bedeutendste Stellung ein. Es wird daher besonders bevorzugt genutzt für die Reinigung von Kohlenwasserstoffgemischen, die Olefine mit drei und/oder vier Kohlenstoffatomen aufweisen.

Das erfindungsgemäße Sorptionsmittel lässt sich besonders vorteilhaft einsetzen zum Reinigen von typischen C₄-Kohlenwasserstoffströme in einem Aufarbeitungszustand unmittelbar vor Umsetzung der darin enthaltenen Butene. Die "Verunreinigungen" umfasst neben den Schwefel enthaltenden Verbindungen auch Basen wie beispielsweise Amine oder Nitrile, die allerdings unter der Nachweisgrenze liegen.

Das Verfahren ist auf solche Gemische besonders gut anwendbar, da es als Gifte für die heterogenen, aluminium-, silicium- oder nickelhaltigen Oligomerisierungskatalysatoren wirkenden Verunreinigungen gut entfernt.

Bei den Verunreinigungen, die erfindungsgemäß aus dem verunreinigten Kohlenwasserstoffgemisch entfernt werden sollen, handelt es sich vorzugsweise um organische Schwefelverbindungen, die in der nachfolgenden Aufarbeitung des Kohlenwasserstoffgemisches als Katalysatorgift wirken. Zu katalysatorschädlichen, organischen Schwefelverbindungen, die in den üblicherweise erhältlichen Rohstoffströmen enthalten sind, gehören insbesondere:
a) Thiole mit der allgemeinen Formel R-SH,
b) Disulfide mit der allgemeinen Formel R-S-S-R',
c) Sulfide mit der allgemeinen Formel R-S-R' und
d) substituierte oder unsubstituierte schwefelhaltige Heterozyklen, wie insbesondere Thiophene und/oder Thiolane.

In den oben angegebenen Strukturformeln können R und R' gleiche oder unterschiedliche Alkyl-, Aryl-, Cycloalkyl-, oder Alkenyl-Reste sein, wobei es sich bei R und R' insbesondere um Methyl-, Ethyl-,
Propyl-, Butyl-, Phenyl-, Cyclohexyl- oder Butenyl-Reste handelt.

Der besondere Vorteil der erfindungsgemäß eingesetzten Sorptionsmaterialien besteht darin, dass es die Verunreinigungen chemisch sorbiert und zwar insbesondere dadurch, dass als Verunreinigung enthaltene Thiole an der Oberfläche des ersten Sorptionsmittels arretiert werden. Zugeführte oder etwaig im ersten Bett gebildete Disulfide werden spätestens an dem zweiten Sorptionsmittel arretiert. Darüber hinaus sorbiert das zweite Sorptionsmittel auch Thiole, die das erste Bett durchbrochen haben. Die Chemiesorption in beiden Betten bewirkt einen besonders hohen Reinigungsgrad, sodass das Kohlenwasserstoffgemisch nahezu vollständig von enthaltenen Thiolen und Disulfiden befreit wird.

Die Chemisorption der Katalysatorgifte ist irreversibel. Aus diesem Grunde können die erfindungsgemäß eingesetzten Sorptionsmittel nicht regeneriert werden. Dies bedeutet, dass stark verunreinigte Kohlenwasserstoffströme die Sorptionsmittel rasch erschöpfen, sodass sie ausgetauscht werden müssen. Im Interesse des wirtschaftlichen Betriebs des Reinigungsverfahrens sollte der Gewichtsanteil der Verunreinigungen in dem verunreinigten Kohlenwasserstoffgemisch bezogen auf dessen Gesamtgewicht kleiner als 0.2 Gew.-% sein. Besonders bevorzugt enthält das verunreinigte Kohlenwasserstoffgemisch weniger als 100 Gew.-ppm und besonders bevorzugt weniger als 10 Gew.-ppm Verunreinigungen, jeweils gerechnet als Schwefel. Bei einem solch geringen Verunreinigungsgrad lässt sich das Sorptionsmittel sehr lange betreiben und ermöglicht zudem eine nahezu restlose Entfernung der Katalysatorgifte. Die Maßeinheit ppm versteht sich wie immer als 10⁻⁶.

Nun weisen die üblichen aus Erdölraffinerien stammenden Rohstoffgemische Schwefelgehalte von weit über 0.2 Gew.-% auf. Aus diesem Grunde ist es erforderlich, das Rohstoffgemisch in einer der sorptiven Reinigung vorgeschalteten Vorreinigungsstufe vorzureinigen. In der Vorreinigungsstufe wird das stärker verunreinigte Rohstoffgemisch unter Erhalt eines Kohlenwasserstoffgemisches vorgereinigt, dessen Verunreinigungsgrad unter 0.2 Gew.-% liegt.

Als Vorreinigungsstufe eignet sich insbesondere die oben beschriebene MEROX®-Wäsche oder eine Thioveretherung, wie sie in der WO2014009148A1 offenbart ist.

Die erfindungsmäße Form der Reinigung eignet sich insbesondere dafür, hinter einer MEROX®-Wäsche als Polizei-Filter in den Strom eingeschaltet zu werden.

Unter einem Polizei-Filter ist in diesem Zusammenhang eine zweite Reinigungsinstanz zu verstehen, die hinter einer ersten Reinigungsinstanz angeordnet ist und deren Funktion darin besteht, von der ersten Reinigungsinstanz nicht erfasste Restmengen der Katalysatorgifte endgültig von nachfolgenden Reaktionsschritten fernzuhalten oder im Falle einer Betriebsstörung in der ersten Instanz eine sofortige Schädigung der nachfolgenden Reaktionsschritte auszuschließen.

Als erste Reinigungsinstanz dient vorzugsweise eine MEROX®-Wäsche, welche die meisten Katalysatorgifte in größeren Mengen vorweg abscheidet. Lediglich die von der MEROX®-Wäsche nicht erfassten Mercaptane und Disulfide werden dann erfindungsgemäß in den beiden Sorptionsbetten zurückgehalten. Da der erfindungsgemäße Polizei-Filter selbst zweistufig ist, weist die gesamte Reinigung inkl. Vorreinigung mindestens drei Stufen auf.

Für den Fall einer Betriebsstörung in der Vorreinigung übernimmt der Polizei-Filter die volle Reinigungsfunktion und schützt die Oligomerisierung vor sofortigen irreversiblen Schäden. Da der Polizei-Filter im normalen Betriebszustand nur eine geringe Menge Adsorbat aufnimmt, darf er kapazitiv deutlich kleiner ausgelegt werden als eine typische zur Vorreinigung verwendete MEROX® -Wäsche. Entsprechend rasch ist er bei einem Störfall erschöpft. Die geeignete Dimensionierung des Polizei-Filters hängt davon ab, wie schnell das anströmende Gemisch umgeleitet werden kann.

Thioether als vergleichsweise unreaktive Substanzen werden in MEROX®-Wäschen praktisch nicht entfernt. Um allzu große Konzentrationen am Eintritt in das Sorptionsbett zu vermeiden, werden sie vorzugsweise an einer geeigneten Stelle im Prozessablauf vor dem Sorptionsbett als Hochsieder in einer Destillation abgetrennt.

In Kombination mit einer Vorreinigungsstufe wie einer MEROX®-Wäsche können beide hier beschriebenen Sorptionsmittel bedenkenlos irreversibel eingesetzt werden. Unter einem irreversiblen Einsatz ist in diesem Zusammenhang zu verstehen, dass keine unmittelbare Regeneration, also Wiedergewinnung des aktiven Sorptionsmittels erfolgt, sobald dieses desaktiviert ist. Dies schließt nicht aus, dass das verbrauchte Sorptionsmittel recycelt wird, indem die darin enthaltenen Metalle, wie insbesondere das Kupfer, metallurgisch wiedergewonnen werden. Bei einer solchen metallurgischen Behandlung geht nämlich die ursprüngliche Zusammensetzung des Sorptionsmittels verloren, sodass in diesem Zusammenhang nicht von einer Regeneration gesprochen werden kann.

Das erfindungsgemäße Verfahren eignet sich grundsätzlich zur Entschwefelung von Kohlenwasserstoffströmen mit drei bis acht Kohlenstoffatomen. Besonders bevorzugt wird es jedoch zur Entgiftung von C₄-Strömen verwendet, die als Crack C4 oder als FCC C4 oder ihre entsprechenden Raffinate bei der Raffinierung von Erdöl anfallen. Bevorzugt erfüllt also das verunreinigte Kohlenwasserstoffgemisch eine der folgenden, sich jeweils zu 100 Gew.-% ergänzenden Spezifikationen A, B, C oder D, wobei sich die angegebenen Gewichtsanteile jeweils auf das Gesamtgewicht des verunreinigten Kohlenwasserstoffgemisches beziehen.

### Spezifikation A:

- Isobutan 15 bis 45 Gew.-%, bevorzugt 25 bis 35 Gew.-%;
- n-Butan 5 bis 18 Gew.-%, bevorzugt 8 bis 10 Gew.-%;
- 1-Buten 5 bis 20 Gew.-%, bevorzugt 12 bis 14 Gew.-%;
- Isobuten 12 bis 25 Gew.-%, bevorzugt 15 bis 20 Gew.-%;
- 2-Butene 9 bis 40 Gew.-%, bevorzugt 20 bis 30 Gew.-%;
- 1.3-Butadien 0 bis 3 Gew.-%, bevorzugt 0.5 bis 0.8 Gew.-%;
- Wasser 0 bis 1 Gew.-%, bevorzugt weniger als 0.1 Gew.-%;
- schwefelhaltige Verunreinigungen weniger als 0.5 Gew.-%, bevorzugt kleiner 0.2 Gew.-%.

### Spezifikation B:

- Isobutan 0.5 bis 15 Gew.-%, bevorzugt 1 bis 7 Gew.-%;
- n-Butan 0.5 bis 20 Gew.-%, bevorzugt 4 bis 7 Gew.-%;
- 1-Buten 9 bis 25 Gew.-%, bevorzugt 10 bis 20 Gew.-%;
- Isobuten 10 bis 35 Gew.-%, bevorzugt 20 bis 30 Gew.-%;
- 2-Butene 3 bis 15 Gew.-%, bevorzugt 5 bis 10 Gew.-%;
- 1.3-Butadien 25 bis 70 Gew.-%, bevorzugt 40 bis 50 Gew.-%;
- Wasser 0 bis 1 Gew.-%, bevorzugt weniger als 0.5 Gew.-%;
- schwefelhaltige Verunreinigungen weniger als 0.5 Gew.-%, bevorzugt kleiner 0.2 Gew.-%.

### Spezifikation C:

- Isobutan 0.5 bis 18 Gew.-%, bevorzugt 1 bis 7 Gew.-%;
- n-Butan 0.5 bis 25 Gew.-%, bevorzugt 4 bis 13 Gew.-%;
- 1-Buten 9 bis 40 Gew.-%, bevorzugt 10 bis 35 Gew.-%;
- Isobuten 10 bis 55 Gew.-%, bevorzugt 20 bis 50 Gew.-%;
- 2-Butene 3 bis 25 Gew.-%, bevorzugt 5 bis 20 Gew.-%;
- 1.3-Butadien 0 bis 5 Gew.-%, bevorzugt weniger als 0.8 Gew.-%;
- Wasser 0 bis 1 Gew.-%, bevorzugt weniger als 0.5 Gew.-%;
- schwefelhaltige Verunreinigungen weniger als 0.5 Gew.-%, bevorzugt kleiner 0.2 Gew.-%.

### Spezifikation D:

- Isobutan 0 bis 20 Gew.-%, bevorzugt 0 bis 5 Gew.-%;
- n-Butan 10 bis 35 Gew.-%, bevorzugt 25 bis 30 Gew.-%;
- 1-Buten 0.2 bis 45 Gew.-%, bevorzugt 3 bis 30 Gew.-%;
- 2-Butene 35 bis 85 Gew.-%, bevorzugt 50 bis 75 Gew.-%;
- Wasser 0 bis 1 Gew.-%, bevorzugt weniger als 0.1 Gew.-%;
- schwefelhaltige Verunreinigungen, weniger als 0.5 Gew.-%, bevorzugt kleiner 0.1 Gew.-%.

Die Spezifikation A beschreibt dabei typisches FCC C4, währenddessen die Spezifikation B typisches Crack C4 beschreibt. Spezifikation C beschreibt ein typisches Raffinat I aus Crack C4. Spezifikation D schließlich beschreibt ein Raffinat III aus FCC oder CC4.

Bei den in den Spezifikationen genannten, schwefelhaltigen Verunreinigungen handelt es sich vorzugweise um Substanzen aus einer der folgenden Substanzklassen:
a) Thiole mit der allgemeinen Formel R-SH;
b) Disulfide mit der allgemeinen Formel R-S-S-R';
c) Sulfide mit der allgemeinen Formel R-S-R';
d) substituierte oder unsubstituierte schwefelhaltige Heterozyklen, wie insbesondere Thiophene und/oder Thiolane;
wobei in den angegebenen Strukturformeln R und R' gleiche oder unterschiedliche Alkyl-, Aryl-, Cycloalkyl-, oder Alkenyl-Reste sein können. Bei R und R' handelt es sich dabei insbesondere um Methyl-, Ethyl-, Propyl-, Butyl-, Phenyl-, Cyclohexyl- oder Butenyl-Reste.

Nachdem das verunreinigte Kohlenwasserstoffgemisch erfindungsgemäß von seinen Katalysatorgiften befreit wurde, kann die übliche Aufarbeitung solcher Gemische erfolgen, ohne dabei eine Vergiftung der stromabwärts eingesetzten Katalysatoren befürchten zu müssen. Zu den typischen Aufarbeitungsschritten, die auf die hier beschriebene Reinigung folgen können, gehören:
a) Extraktion von im Kohlenwasserstoffgemisch enthaltenden 1.3-Butadien;
b) Selektivhydrierung von im Kohlenwasserstoffgemisch enthaltenden Diolefinen und/oder Acetylenen zu Olefinen;
c) Oligomerisierung von im Kohlenwasserstoffgemisch enthaltenden Olefinen zu entsprechenden Oligomeren;
d) Destillative Abtrennung von im Kohlenwasserstoffgemisch enthaltenden 1-Buten und/oder Isobutan, insbesondere mit dem Zweck, 1-Buten und/oder Isobutan in hoher Reinheit zu erhalten;
e) Entfernung von im Kohlenwasserstoffgemisch enthaltenden Isobuten durch Umwandlung des Isobutens mit Wasser zu tert.-Butanol und/oder mit Methanol zu Methyl-tert.-Butylether;
f) Dehydrierung von im Kohlenwasserstoffgemisch enthaltenden Butanen zu Butenen;
g) oxidative Dehydrierung von im Kohlenwasserstoffgemisch enthaltenden Butenen zu Butadien;
h) Alkylierung von im Kohlenwasserstoffgemisch enthaltenden von n-Buten mit ebenfalls enthaltendem Isobutan;
i) Oxidation von im Kohlenwasserstoffgemisch enthaltenden Kohlenwasserstoffe mit vier Kohlenstoffatomen zur Herstellung von Maleinsäureanhydrid.

Selbstverständlich müssen nicht alle aufgezählten Aufarbeitungsschritte a) bis i) durchgeführt werden, es können auch nur einzelne durchgeführt werden. Auch ist die aufgezählte Reihenfolge nicht bindend.

Darüber hinaus können einzelne der aufgezählten Aufarbeitungsschritte auch vor der erfindungsgemäßen Reinigung angeordnet sein, sofern diese nicht gegen die Katalysatorgifte empfindlich sind. Zumindest eine Nickel-katalysierte Oligomerisierung sollte mit dem erfindungsgemäßen Sorptionsmittel geschützt werden, da organische Schwefelverbindungen selbst in geringsten Konzentrationen Nickel-Katalysatoren vergiften.

Sofern das eingesetzte Kohlenwasserstoffgemisch auch mit Wasser verunreinigt ist, empfiehlt es sich das mit Wasser verunreinigte Kohlenwasserstoffgemisch vor Eintritt in das Reinigungsbett von Wasser zu befreien, also zu trocknen. Die Abtrennung des Wassers geschieht aus folgender Motivation: Da homogen gelöstes Wasser im Gemisch die Wirkung des Sorptionsmittels etwas abschwächt, wird der Strom vorzugsweise vor Eintritt in das Reinigungsbett getrocknet, beispielsweise durch eine Azeotropdestillation (Trocknungsdestillation).

### Beispiele

### Erster Versuch: Entfernung von Ethanthiol gemäß der Erfindung

Als erstes Sorptionsmittel wird ein von der Clariant AG erworbener Feststoff eingesetzt, der als Methanol-Katalysator verwendbar ist. Das Sorptionsmittel enthält ca. 42 Gew-% CuO, ca. 44 Gew-% ZnO, ca. 12 Gew- % Al₂O₃ und ca. 2 Gew-% Graphit und liegt in Form von Tabletten (5 x 3 mm) vor. Die spezifische Kupferoxidoberfläche beträgt, gemessen mittels Stickstoff-Sorption, 100m² pro g Kupferoxidgehalt. In ein Reaktionsrohr mit 1 cm Durchmesser werden 29 g Sorptionsmittel gefüllt. Die Schüttdichte beträgt ca. 1.2 kg/dm³.

Als zweites Sorptionsmittel wird der handelsübliche Katalysator H 10126 rs der Evonik Industries AG, enthaltend ca. 45 Gew-% Ni, ca. 28 Gew-% ZnO, ca. Gew-25 % SiO₂ und ca. 2 Gew-% Graphit, in Form von zylindischen Kalotten-Tabletten 5 x 5 mm, mit einer mittels Wasserstoff-Chemisorption gemessenen Nickeloberfläche von 9 m²/g in ein Reaktionsrohr mit 1 cm Durchmesser und 23 g Inhalt gefüllt. Die Schüttdichte beträgt ca. 1.15 kg/dm³. Das Adsorptionsmittel wird im Stickstoff-Wasserstoffstrom bei ca. 220°C reaktiviert, wobei je nach Wärmetönung zunächst 1 Vol- % Wasserstoff, am Ende 50 Vol-% Wasserstoff zugemischt werden. Danach wird das Katalysatorbett im Stickstoffstrom abgekühlt.

Die beiden Rohre werden dann in Reihe geschaltet, wobei zuerst das Rohr mit dem Cu-haltigen Sorptionsmittel und dann das Rohr mit dem Ni-haltige Sorptionsmittel mit verunreinigtem Feed in Berührung kommen. Die Betten werden durch Beheizung der Rohrwände auf eine Temperatur von 100° C gebracht und bei einem Druck von 24 bar mit einem Gemisch durchströmt, das ca. 38 Gew-% 1-Buten, ca. 23 Gew-% trans-2-Buten, ca. 15 Gew-% cis-2-Buten und ca. 23 Gew-% n-Butan enthält. Als Verunreinigung enthält das Material im Mittel 5 mg/kg an Schwefel in Form von Ethanthiol. Die Belastung der Adsorberbetten beträgt 370 g/h, der Schwefeleintrag also etwa 1.85 mg/h.

Der Schwefel wird ausweislich der Analysen zunächst nahezu quantitativ aus dem Gemisch entfernt (Tabelle 1). Der Durchbruch der gesamten Sequenz erfolgt nach etwa 338 Stunden. Die beiden Reinigungsbetten haben zu diesem Zeitpunkt gemeinsam insgesamt ca. 0.73 g Schwefel aufgenommen, entsprechend einer Aufnahme von 1.4 Gew.-% bezogen auf die frisch eingefüllten Sorptionsmittel.

Die Austragswerte der einzelnen C₄-Komponenten, insbesondere 1-Buten, blieben gegenüber den entsprechenden Zulaufwerten während der gesamten Versuchszeit unverändert.

Nach dem Ende dieses Versuchs wird das zweite Bett mit warmem Stickstoff gespült und anschließend mit kaltem Stickstoff-Luft-Gemisch vorsichtig oxidiert, bis in reiner Luft keine wesentliche Wärmetönung mehr erfolgt.

Beide Sorptionsmittel können im Wesentlichen unversehrt und mit noch hinreichender Festigkeit entnommen werden.

Die Ergebnisse des Versuchs sind in Tabelle 1 niedergelegt.

**Tabelle 1: Ergebnisse aus Versuch 1**

| Mittlerer S-Gehalt [Gew.-%] Zulauf | Mittlerer S-Gehalt [Gew.-%] nach dem Cu-Bett bis 338 h | Mittlerer S-Gehalt [Gew.-%] Austrag bis 338 h | Mittlere Abnahme S [Gew.-%] in Austrag gegenüber Zulauf bis 338 h |
|---|---|---|---|
| 0.00051 | 0.00012* | 0.00003 | 94 |

| | | | |
|---|---|---|---|
| *nahezu ausschließlich in Form von Diethyldisulfid | | | |

### Zweiter Versuch: Entfernung von Methanthiol gemäß der Erfindung

Die verwendeten Sorptionsmittel und der Versuchsaufbau entsprechen dem ersten Versuch.

Analog Versuch 1 wird als Verunreinigung 5 mg/kg an Schwefel überwiegend in Form von Methanthiol zugeführt. Die Belastung der beiden Reinigungsbetten, deren Füllmenge 28 g und 23 g beträgt, liegt bei 420 g/h, der Schwefeleintrag also etwa 2.1 mg/h. Die Kontakttemperatur wurde auf 100°C eingestellt.

Der Schwefel wird ausweislich der Analysen zunächst nahezu quantitativ aus dem Gemisch entfernt. Der Durchbruch nach dem kombinierten Reinigungsbett erfolgt nach etwa 600 Stunden. Die Reinigungsbetten haben zu diesem Zeitpunkt insgesamt ca. 0.9 g Schwefel aufgenommen, entsprechend einer mittleren Aufnahme von 1.7 Gew.-% bezogen auf die Summe der frisch eingefüllten Sorptionsmittel.

Die Austragswerte der einzelnen C₄-Komponenten, insbesondere 1-Buten, blieben gegenüber den entsprechenden Zulaufwerten während der gesamten Versuchszeit unverändert. Mithin wurde kein 1-Buten in Richtung 2-Buten isomerisiert, sodass der Wert des Einsatzgemisches nicht gemindert wurde.

Nach dem Ende dieses Versuchs wird das zweite Bett mit warmem Stickstoff gespült und anschließend mit kaltem Stickstoff-Luft-Gemisch vorsichtig oxidiert, bis in reiner Luft keine wesentliche Wärmetönung mehr erfolgt.

Beide Sorptionsmittel können im Wesentlichen unversehrt und mit noch hinreichender Festigkeit entnommen werden.

Die Versuchsergebnisse sind in Tabelle 2 dargestellt.

**Tabelle 2: Ergebnisse aus Versuch 2**

| Mittlerer S-Gehalt [Gew.-%] Zulauf | Mittlerer S-Gehalt [Gew.-%] nach dem Cu-Bett bis 600 h | Mittlerer S-Gehalt [Gew.-%] Austrag bis 600 h | Mittlere Abnahme S [Gew.-%] in Austrag gegenüber Zulauf bis 600 h |
|---|---|---|---|
| 0.00051 | 0.00011* | 0.00005 | 90 |

| | | | |
|---|---|---|---|
| *überwiegend in Form von Dimethyldisulfid | | | |

### Dritter Versuch: Entfernung von Diethyldisulfid gemäß der Erfindung

Das verwendete Sorptionsmittel und der Versuchsaufbau entsprechen dem ersten und zweiten Versuch.

Analog Versuch 1 wird als Verunreinigung 1 mg/kg an Schwefel überwiegend in Form von Diethyldisulfid zugeführt. Die Belastung der Reinigungsbetten, die 28 g und 21 g der Sorptionsmittel enthalten, beträgt 380 g/h, der Schwefeleintrag also etwa 0.4 mg/h. Die Betriebstemperatur beträgt 100°C.

Der Schwefel wird ausweislich der Analysen zunächst nahezu quantitativ aus dem Gemisch entfernt. Der Durchbruch nach dem kombinierten Reinigungsbett erfolgt nach etwa 630 Stunden. Die Reinigungsbetten haben zu diesem Zeitpunkt insgesamt ca. 0.3 g Schwefel aufgenommen, entsprechend einer mittleren Aufnahme von 0.6 Gew.-% bezogen auf das frisch eingefüllte Sorptionsmittel.

Die Austragswerte der einzelnen C₄-Komponenten, insbesondere 1-Buten, blieben gegenüber den entsprechenden Zulaufwerten während der gesamten Versuchszeit unverändert. Mithin wurde kein 1-Buten in Richtung 2-Buten isomerisiert, sodass der Wert des Einsatzgemisches nicht gemindert wurde.

Nach dem Ende dieses Versuchs wird das zweite Bett mit warmem Stickstoff gespült und anschließend mit kaltem Stickstoff-Luft-Gemisch vorsichtig oxidiert, bis in reiner Luft keine wesentliche Wärmetönung mehr erfolgt.

Beide Sorptionsmittel können im Wesentlichen unversehrt und mit noch hinreichender Festigkeit entnommen werden.

Die Versuchsergebnisse sind in Tabelle 3 dargestellt.

**Tabelle 3: Ergebnisse aus Versuch 3**

| Mittlerer S-Gehalt [Gew.-%] Zulauf | Mittlerer S-Gehalt [Gew.-%] nach dem Cu-Bett bis 630 h | Mittlerer S-Gehalt [Gew.-%] Austrag bis 630 h | Mittlere Abnahme S [Gew.-%] in Austrag gegenüber Zulauf bis 630 h |
|---|---|---|---|
| 0.00011 | 0.00002* | 0.000003 | 97 |

| | | | |
|---|---|---|---|
| *überwiegend in Form von Diethyldisulfid | | | |

### Vierter Versuch: Entfernung von Ethanthiol gemäß der Erfindung

Die verwendeten Sorptionsmittel und der Versuchsaufbau entsprechen dem ersten Versuch.

Analog Versuch 1 wird als Verunreinigung 5 mg/kg an Schwefel überwiegend in Form von Ethanthiol zugeführt. Die Belastung der beiden Reinigungsbetten, deren Füllmenge 28 g und 21,5 g beträgt, liegt bei 385 g/h, der Schwefeleintrag also etwa 1.9 mg/h. Die Kontakttemperatur wurde auf 80°C eingestellt.

Der Schwefel wird ausweislich der Analysen zunächst nahezu quantitativ aus dem Gemisch entfernt. Der Durchbruch nach dem kombinierten Reinigungsbett erfolgt nach etwa 535 Stunden. Die Reinigungsbetten haben zu diesem Zeitpunkt insgesamt ca. 1.0 g Schwefel aufgenommen, entsprechend einer mittleren Aufnahme von 2.0 Gew.-% bezogen auf die Summe der frisch eingefüllten Sorptionsmittel.

Die Austragswerte der einzelnen C₄-Komponenten, insbesondere 1-Buten, blieben gegenüber den entsprechenden Zulaufwerten während der gesamten Versuchszeit unverändert. Mithin wurde kein 1-Buten in Richtung 2-Buten isomerisiert, sodass der Wert des Einsatzgemisches nicht gemindert wurde.

Nach dem Ende dieses Versuchs wird das zweite Bett mit warmem Stickstoff gespült und anschließend mit kaltem Stickstoff-Luft-Gemisch vorsichtig oxidiert, bis in reiner Luft keine wesentliche Wärmetönung mehr erfolgt.

Beide Sorptionsmittel können im Wesentlichen unversehrt und mit noch hinreichender Festigkeit entnommen werden.

Die Versuchsergebnisse sind in Tabelle 4 dargestellt.

**Tabelle 4: Ergebnisse aus Versuch 4**

| Mittlerer S-Gehalt [Gew.-%] Zulauf | Mittlerer S-Gehalt [Gew.-%] nach dem Cu-Bett bis 535 h | Mittlerer S-Gehalt [Gew.-%] Austrag bis 535 h | Mittlere Abnahme S [Gew.-%] in Austrag gegenüber Zulauf bis 535 h |
|---|---|---|---|
| 0.00052 | 0.00020* | 0.00006 | 88 |

| | | | |
|---|---|---|---|
| *nahezu ausschließlich in Form von Diethyldisulfid | | | |

### Fünfter Versuch: Entfernung von Dimethyldisulfid mit Hilfe von Zeolithen (nicht erfindungsgemäß)

Es wird ein Sorptionsmittel nach EP0354316 hergestellt. Es basiert auf einem Zeolithen des Typs X basiert und enthält lediglich 10 Gew.-% Cu. Die zwei mit jeweils 50 g des Materials gefüllten Rohre werden in Reihe geschaltet. Die Betten werden durch Beheizung der Rohrwände auf eine Temperatur von 120°C gebracht und bei einem Druck von 30 bar mit einem flüssigen Gemisch durchströmt, das ca. 33 Gew-% 1-Buten, ca. 23 Gew-% trans-2-Buten, ca. Gew-15 % cis-2-Buten und ca. 27 Gew-% n-Butan enthält. Als Verunreinigung enthält das Material im Mittel 2.0 mg/kg an Schwefel überwiegend in Form von Dimethyldisulfid. Die Belastung der Reinigungsbetten beträgt 375 g/h, der Schwefeleintrag also etwa 0.75 mg/h.

Der Schwefel wird ausweislich der Analysen zunächst nahezu quantitativ aus dem Gemisch entfernt. Der Durchbruch nach dem zweiten Reinigungsbett (Austrag 2) erfolgt nach etwa 96 Stunden. Die Reinigungsbetten haben zu diesem Zeitpunkt insgesamt ca. 0.07 g Schwefel aufgenommen, entsprechend einer mittleren Aufnahme von 0.07 Gew.-% bezogen auf das frisch eingefüllte Sorptionsmittel.

Mit dem nicht erfindungsgemäßen Material ist demnach nur für sehr kurze Zeit eine deutliche Entschwefelung zu erreichen, wobei der Materialeinsatz in keinem Verhältnis zur Reinigungsleistung steht.

Die Ergebnisse sind in Tabelle 5 dargestellt.

**Tabelle 5: Ergebnisse aus Versuch 5**

| Mittlerer S-Gehalt [Gew.-%] Zulauf | Mittlerer S-Gehalt [Gew.-%] Austrag 1 bis 48 h | Mittlerer S-Gehalt [Gew.-%] Austrag 2 bis 96 h | Mittlere Abnahme S [Gew.-%] in Austrag 2 gegenüber Zulauf bis 96 h |
|---|---|---|---|
| 0.00020 | 0.000005 | 0.000005 | 97 |

Die Versuche belegen, dass die erfindungsgemäß eingesetzte Kopplung zweier Sorptionsmittel die folgenden Eigenschaften aufweist:
- Es bindet den Schwefel aus diversen Schwefelverbindungen nahezu vollständig;
- Mögliche gebildete Nebenprodukte des ersten Festbettes sowie im ersten Sorptionsbett nicht vollständig absorbierte Schwefel-Komponenten werden im zweiten Sorptionsbett nahezu vollständig aufgenommen;
- Es benötigt keine zusätzlichen Betriebsstoffe;
- Es benötigt keine periodischen Reinigungs- und Desorptionsströme, da es sich um ein irreversibles Sorptionsmittel handelt;
- Es kann in einem einfachen Behälter untergebracht werden, der einfach vom dem Gemisch durchströmt wird, vorzugsweise bei leicht erhöhter Temperatur, wie sie typischerweise oft ohnehin für die Speisung von nachfolgenden Reaktoren erforderlich ist;
- Es verursacht praktisch keine Nebenreaktionen der Olefine wie Oligomerisierung, Isomerisierung und Hydrierung und damit auch keine Wertverluste;
- Es setzt keinerlei Stoffe in Konzentrationen frei, die irgendeinen Einfluss auf die nachfolgenden Verarbeitungsstufen haben;
- Es ist angesichts der bei typischen Schwefelkonzentrationen unter 5 ppmw und eine mittlere Kapazität von mindestens 0.5 Gew.-% Schwefel langen Lebensdauer sehr kostengünstig im Betrieb, auch wenn es nicht direkt regeneriert werden kann, sondern nach Erschöpfung der Kapazität nur noch einer rohstofflichen Verwertung zugeführt werden kann;

## Patentansprüche

1. Verfahren zur Reinigung von Kohlenwasserstoffgemischen, bei welchem ein verunreinigtes Kohlenwasserstoffgemisch enthaltend Olefine mit drei bis acht Kohlenstoffatomen durch Kontakt mit einem ersten festen Sorptionsmittel zumindest teilweise von Verunreinigungen befreit wird, wobei sich das Kohlenwasserstoffgemisch während des Kontakts mit dem ersten festen Sorptionsmittel ausschließlich im flüssigen Zustand befindet,
**dadurch gekennzeichnet,**
**dass** das erste feste Sorptionsmittel die folgende, sich zu 100 Gew.-% ergänzende Zusammensetzung aufweist:
• Kupferoxid: 10 bis 60 Gew.-%;
• Zinkoxid: 10 bis 60 Gew.-%;
• Aluminiumoxid: 10 bis 30 Gew.-%;
• sonstige Stoffe: 0 bis 5 Gew.-%;
**dass** das Kohlenwasserstoffgemisch nach dem Kontakt mit dem ersten festen Sorptionsmittel mit einem vom ersten festen Sorptionsmittel verschiedenen, zweiten festen Sorptionsmittel in Kontakt gebracht wird, welches die folgende, sich zu 100 Gew.-% ergänzende Zusammensetzung aufweist:
• Nickel: 15 Gew.-% bis 65 Gew.-%;
• Zinkoxid: 0 Gew.-% bis 40 Gew.-%;
• Siliciumdioxid: 5 Gew.-% bis 75 Gew.-%;
• Graphit: 0 Gew.-% bis 5 Gew.-%;
• sonstige Komponenten weniger als 40 Gew.-%;
wobei sich das Kohlenwasserstoffgemisch auch während des Kontakts mit dem zweiten festen Sorptionsmittel ausschließlich im flüssigen Zustand befindet.

2. Verfahren nach Anspruch 1,
**dadurch gekennzeichnet,**
**dass** das zweite feste Sorptionsmittel eine metallische Nickeloberfläche von größer als 3 m²/g aufweist, bezogen auf das Gesamtgewicht an Nickel in dem zweiten Sorptionsmittel.

3. Verfahren nach Anspruch 1 oder 2,
**dadurch gekennzeichnet,**
**dass** das erste feste Sorptionsmittel in einem ersten Bett angeordnet ist und das zweite feste Sorptionsmittel in einem stromabwärts hinter dem ersten Bett gelegenen zweiten Bett angeordnet ist, dergestalt, dass das Kohlenwasserstoffgemisch nacheinander durch das erste und sodann durch das zweite Bett strömt.

4. Verfahren nach einem der vorhergehenden Ansprüchen,
**dadurch gekennzeichnet,**
**dass** der Kontakt mit beiden Sorptionsmitteln in Abwesenheit von Wasserstoff erfolgt.

5. Verfahren nach einem der vorhergehenden Ansprüchen,
**dadurch gekennzeichnet,**
**dass** der Kontakt mit beiden Sorptionsmitteln unter den folgenden Bedingungen erfolgt, wobei beim ersten Kontakt und beim zweiten Kontakt unterschiedliche oder identische Bedingungen gewählt sein können:
• Temperatur zwischen 10°C und 150°C;
• Druck zwischen 0.5 und 3.5 MPa;
• Raum/Zeit-Belastung (weight hour space velocity - WHSV) zwischen 0.5 h⁻¹ und 15 h⁻¹

6. Verfahren nach einem der vorhergehenden Ansprüche,
**dadurch gekennzeichnet,**
**dass** das verunreinigte Kohlenwasserstoffgemisch mindestens eine Verunreinigung aus einer der folgenden Substanzklassen enthält:
a) Thiole mit der allgemeinen Formel R-SH
wobei R ein Alkyl-, Aryl-, Cycloalkyl- oder ein Alkenyl-Rest sein kann, wobei es sich bei R insbesondere ein Methyl-, Ethyl-, Propyl-, Butyl-, Phenyl-, Cyclohexyl- oder Butenyl-Rest handelt;
b) Disulfide mit der allgemeinen Formel R-S-S-R',
wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
c) Sulfide mit der allgemeinen Formel R-S-R'
wobei R und R' gleiche oder unterschiedliche Alkyl-, Aryl-, Cycloalkyl-, oder Alkenyl-Reste sein können, wobei es sich bei R und R' insbesondere um Methyl-, Ethyl-, Propyl-, Butyl-, Phenyl-, Cyclohexyl- oder Butenyl-Reste handelt;
d) Subtitutierte oder unsubstituierte schwefelhaltige Heterocyclen, insbesondere Thiophene und/oder Thiolane.

7. Verfahren nach einem der vorhergehenden Ansprüchen,
**dadurch gekennzeichnet,**
**dass** Gewichtsanteil der Verunreinigungen in dem verunreinigten Kohlenwasserstoffgemisch bezogen auf dessen Gesamtgewicht kleiner ist als 0.2 Gew.-%; besonders bevorzugt, dass er unter 100 Gew.-ppm liegt und ganz besonders bevorzugt, dass er unter 10 Gew.-ppm liegt.

8. Verfahren nach einem der vorhergehenden Ansprüche, **dadurch gekennzeichnet, dass** das verunreinigte Kohlenwasserstoffgemisch aus einer Vorreinigungsstufe erhalten wird, welche ein stärker verunreinigtes Rohstoffgemisch unter Erhalt des verunreinigten Kohlenwasserstoffgemisches vorreinigt.

9. Verfahren nach Anspruch 8,
**dadurch gekennzeichnet,**
**dass** beide Sorptionsmittel irreversibel eingesetzt werden.

10. Verfahren nach einem der vorhergehenden Ansprüche,
**dadurch gekennzeichnet,**
**dass** das verunreinigte Kohlenwasserstoffgemisch eine der folgenden, sich jeweils zu 100 Gew.-% ergänzende Spezifikationen A, B, C oder D erfüllt, wobei sich die angegebenen Gewichtsanteile jeweils auf das Gesamtgewicht des verunreinigten Kohlenwasserstoffgemisches beziehen.
Spezifikation A:
• Isobutan 15 bis 45 Gew.-%, bevorzugt 25 bis 35 Gew.-%;
• n-Butan 5 bis 18 Gew.-%, bevorzugt 8 bis 10 Gew.-%;
• 1-Buten 5 bis 20 Gew.-%, bevorzugt 12 bis 14 Gew.-%;
• Isobuten 12 bis 25 Gew.-%, bevorzugt 15 bis 20 Gew.-%;
• 2-Butene 9 bis 40 Gew.-%, bevorzugt 20 bis 30 Gew.-%;
• 1.3-Butadien 0 bis 3 Gew.-%, bevorzugt 0.5 bis 0.8 Gew.-%;
• Wasser 0 bis 1 Gew.-%, bevorzugt weniger als 0.1 Gew.-%;
• schwefelhaltige Verunreinigungen weniger als 0.5 Gew.-%, bevorzugt kleiner 0.2 Gew.-%.
Spezifikation B:
• Isobutan 0.5 bis 15 Gew.-%, bevorzugt 1 bis 7 Gew.-%;
• n-Butan 0.5 bis 20 Gew.-%, bevorzugt 4 bis 7 Gew.-%;
• 1-Buten 9 bis 25 Gew.-%, bevorzugt 10 bis 20 Gew.-%;
• Isobuten 10 bis 35 Gew.-%, bevorzugt 20 bis 30 Gew.-%;
• 2-Butene 3 bis 15 Gew.-%, bevorzugt 5 bis 10 Gew.-%;
• 1.3-Butadien 25 bis 70 Gew.-%, bevorzugt 40 bis 50 Gew.-%;
• Wasser 0 bis 1 Gew.-%, bevorzugt weniger als 0.5 Gew.-%;
• schwefelhaltige Verunreinigungen weniger als 0.5 Gew.-%, bevorzugt kleiner 0.2 Gew.-%.
Spezifikation C:
• Isobutan 0.5 bis 18 Gew.-%, bevorzugt 1 bis 7 Gew.-%;
• n-Butan 0.5 bis 25 Gew.-%, bevorzugt 4 bis 13 Gew.-%;
• 1-Buten 9 bis 40 Gew.-%, bevorzugt 10 bis 35 Gew.-%;
• Isobuten 10 bis 55 Gew.-%, bevorzugt 20 bis 50 Gew.-%;
• 2-Butene 3 bis 25 Gew.-%, bevorzugt 5 bis 20 Gew.-%;
• 1.3-Butadien 0 bis 5 Gew.-%, bevorzugt weniger als 0.8 Gew.-%;
• Wasser 0 bis 1 Gew.-%, bevorzugt weniger als 0.5 Gew.-%;
• schwefelhaltige Verunreinigungen weniger als 0.5 Gew.-%, bevorzugt kleiner 0.2 Gew.-%.
Spezifikation D:
• Isobutan 0 bis 20 Gew.-%, bevorzugt 0 bis 5 Gew.-%;
• n-Butan 10 bis 35 Gew.-%, bevorzugt 25 bis 30 Gew.-%;
• 1-Buten 0.2 bis 45 Gew.-%, bevorzugt 3 bis 30 Gew.-%;
• 2-Butene 35 bis 85 Gew.-%, bevorzugt 50 bis 75 Gew.-%;
• Wasser 0 bis 1 Gew.-%, bevorzugt weniger als 0.1 Gew.-%;
• schwefelhaltige Verunreinigungen weniger als 0.5 Gew.-%, bevorzugt kleiner 0.1 Gew.-%.

11. Verfahren nach einem der vorhergehenden Ansprüche,
**dadurch gekennzeichnet,**
**dass** das zumindest teilweise von Verunreinigungen befreite Kohlenwasserstoffgemisch zumindest einem der nachfolgend aufgezählten Aufarbeitungsschritte unterworfen wird:
a) Extraktion von im Kohlenwasserstoffgemisch enthaltenden 1.3-Butadien;
b) Selektivhydrierung von im Kohlenwasserstoffgemisch enthaltenden Diolefinen und/oder Acetylenen zu Olefinen;
c) Oligomerisierung von im Kohlenwasserstoffgemisch enthaltenden Olefinen zu entsprechenden Oligomeren;
d) Destillative Abtrennung von im Kohlenwasserstoffgemisch enthaltenden 1-Buten und/oder Isobutan, insbesondere mit dem Zweck, 1-Buten und/oder Isobutan in hoher Reinheit zu erhalten;
e) Entfernung von im Kohlenwasserstoffgemisch enthaltenden Isobuten durch Umwandlung des Isobutens mit Wasser zu tert.-Butanol und/oder mit Methanol zu Methyl-tert.-Butylether;
f) Dehydrierung von im Kohlenwasserstoffgemisch enthaltenden Butanen zu Butenen;
g) oxidative Dehydrierung von im Kohlenwasserstoffgemisch enthaltenden Butenen zu Butadien;
h) Alkylierung von im Kohlenwasserstoffgemisch enthaltenden von n-Buten mit ebenfalls enthaltendem Isobutan;
i) Oxidation von im Kohlenwasserstoffgemisch enthaltenden Kohlenwasserstoffe mit vier Kohlenstoffatomen zur Herstellung von Maleinsäureanhydrid.
